# EUROPEAN PATENT SPECIFICATION

(11) **EP 2 756 763 B1**
(45) Date of publication and mention of the grant of the patent: **13.12.2017**
(21) Application number: 12830976.2
(22) Date of filing: 17.09.2012
(51) Int. Cl.: A23L 27/30, A23L 33/125, A23L 33/20, A61K 31/715, A61K 31/7004, A23L 29/30

(54) **SWEETENER COMPOSITION FOR ALLEVIATING DIABETES, CONTAINING SLOWLY DIGESTIBLE INGREDIENT**
SÜSSUNGSMITTELZUSAMMENSETZUNG ZUR LINDERUNG VON DIABETES MIT EINEM LANGSAM VERDAULICHEN BESTANDTEIL
UTILISATION D'UNE COMPOSITION ÉDULCORANTE CONTENANT UN AGENT À DIGESTION LENTE POUR RÉDUIRE LE DIABÈTE

(30) Priority: 15.09.2011 KR 20110092801
(43) Date of publication of application: 23.07.2014
(73) Proprietor: CJ CheilJedang Corporation, Jung-gu Seoul 100-749 (KR)
(72) Inventor: KIM, Young Jae, Seoul 150-072 (KR); PARK, Jin Hee, Gyeonggi-do 411-410 (KR); KIM, Min Hae, Incheon 407-050 (KR); KIM, Seong Bo, Seoul 120-830 (KR); HWANG, Se Hee, Seoul 100-014 (KR); LEE, Young Mi, Gyeonggi-do 420-836 (KR)
(74) Representative: Nevant, Marc
(86) International application number: PCT/KR2012/007425
(87) International publication number: WO 2013/039365

(56) References cited:
- EP-A1- 1 864 669
- JP-A- 2005 213 227
- JP-A- 2010 018 528
- KR-A- 20060 126 999
- KR-A- 20090 077 072
- KR-A- 20100 022 478
- US-A1- 2010 204 346
- MATSUO T ET AL: "Effects of dietary D-psicose on diurnal variation in plasma glucose and insulin concentrations of rats", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 70, no. 9, 1 September 2006 (2006-09-01), pages 2081-2085, XP003022571, ISSN: 0916-8451, DOI: 10.1271/BBB.60036

## Description

### Technical Field

The present invention relates to a sweetener composition for use in alleviating diabetes containing D-psicose, a digestion-resistant maltodextrin and rebaudioside A.

### Background Art

Sugar contains sucrose as a main ingredient and is one of representative sweeteners exhibiting a sweet taste upon being added to food. Sugar has an outstanding sweetness and thus has been considered as one of the most preferred sweeteners which are added to various foods and processed foods to improve the food taste and stimulate appetite. However, recently, as the harmful effects of sugar have been revealed, problems concerning the use thereof have been reported. Specifically, excessive sugar intake is a major cause of tooth decay as well as various lifestyle related diseases such as obesity, diabetes, and the like. For these reasons, there is a global demand for an alternative sugar as a replacement for sugar.

Diabetes is a class of metabolic diseases caused by a lack of secreted insulin or when secreted insulin does not work effectively. Diabetes is characterized by high blood glucose levels, which causes various symptoms and leads to discharge of glucose in the urine. There are two types of diabetes - Type 1 and Type 2. Type 1 diabetes is called "juvenile diabetes" and results from the body's failure to produce insulin. In Type 2 diabetes, not enough insulin is produced. Type 2 diabetes results from insulin resistance, a condition in which cells fail to burn glucose effectively due to a weakened insulin response, raising blood glucose levels. It is known that Type 2 diabetes is greatly affected by environmental factors, such as high caloric, high fat and high protein diet, lack of exercise, stress and the like. In addition, it is known that diabetes can also be caused by defects in specific genes or pancreatic surgery, infection, drugs, and the like. In diabetics, although hyperglycemic symptoms are generally the most pronounced and noticeable, hypoglycemic symptoms can be just as fatal as hyperglycemic symptoms. In a person having normal blood glucose levels, the person generally exhibits hypoglycemic symptoms when fasting blood glucose level drop to 55 mg/dL or less. However, it is known that diabetic patients may exhibit hypoglycemic symptoms even if patients have blood glucose levels higher than 70 mg/dL. Accordingly, unconditional provision of a sweetener having low calories or provision of a sweetener having an ability to inhibit the absorption of sugars may lead to unexpected negative reactions owing to an excessively decreased blood glucose level in diabetic patients.

In this context, there is a strong need for an improved sweetener which has a suitable sweetness to replace sugars and low calories, thereby preventing excessive sugar intake by simply inhibiting the absorption of sugars.

D-psicose is an epimer of D-fructose and is a sub-category of functional sugars known as rare sugars. It is known that D-psicose has a high degree of sweetness equivalent to about 60% to 70% that of sugar and has close to zero calories, and thus is effective in preventing or treating adult diseases such as obesity and the like. In addition, D-psicose is also known to have efficacy to prevent and treat diabetes since D-psicose is able to inhibit the absorption of sugars such as glucose, D-fructose and the like. Furthermore, D-psicose is known to have an excellent solubility and thus takes keen attention for application to foods.

D-psicose has a relatively good sweetness, but has a relatively low sweetness as compared with sugar. In this regard, the use of D-psicose alone as a sweetener for food additives cannot satisfy consumers accustomed to the taste of sugar, thereby hindering market acceptance. In order to overcome such problems stemming from the use of D-psicose alone, namely, in order to use D-psicose alone while attaining a sweetness satisfying general consumers, it is inevitable to increase the amount of D-psicose, which can provide excessive thick feeling to foods utilizing D-psicose, thereby causing a deterioration in the texture of foods.

On the other hand, digestion-resistant maltodextrin is a class of dietary fiber (polysaccharides). As can be seen from its name, the digestion-resistant maltodextrin is a dietary fiber which is indigestible in the human body, and is characterized by having a high molecular weight carbohydrate structure having a degree of polymerization higher than general maltodextrins.

As the prior art related to the present invention, there are Korea Patent Publication No. 10-2011-0035805A (published on April 6, 2011), Korea Patent No. 10-0815212 B1 (published on March 19, 2008), Korean Patent No. 10-0910081 B1 (published on July 30, 2009), JP-A-2010 018528, US 2010 0204346 and EP-A-1864669.

### Disclosure

### Technical Problem

The present disclosure is aimed at providing a sweetener composition for alleviating diabetes containing D-psicose, a digestion-resistant maltodextrin and rebaudioside A as active ingredients.

The inventors of the present invention have found that, when D-psicose which is a low calorie sweetener and known to have efficacy in preventing and alleviating diabetes is used alone, D-psicose cannot effectively perform a role as a sweetener due to declined sweetness. For this reason, various attempts have been made to produce a sweetener having an improved sweetness by mixing D-psicose with other sugars or sugar alcohols having an excellent sweetness such as tagatose, xylose, xylitol, and the like. However, it was found that such mixing can excessively inhibit digestion and absorption of sugars introduced into the body, and thus can cause an excessively low level of blood glucose, which in turn leads to side effects fatal to diabetic patients.

The inventors have developed a sweetener composition for alleviating diabetes using D-psicose, a digestion-resistant maltodextrin and rebaudioside A in combination, which may provide advantages including slow digestion of glucose in the body, allowing blood glucose levels to elevate gradually after meals, thereby preventing hyperglycemic symptoms in which the blood glucose level in diabetic patients is sharply increased, while preventing hypoglycemic symptoms fatal to diabetic patients by supplying sugars slowly but consistently and appropriately.

### Technical Solution

In accordance with one embodiment of the present disclosure a sweetener composition for alleviating diabetes contains D-psicose, a digestion-resistant maltodextrin and rebaudioside A as active ingredients.

In accordance with another embodiment of the present disclosure the digestion-resistant maltodextrin may be present in an amount of 0.01 to 200 times the weight of D-psicose, and rebaudioside A may be present in an amount of 0.001 to 2 times the weight of D-psicose.

The invention is defined by the claims.

### Advantageous Effects

Using D-psicose, a digestion-resistant maltodextrin and rebaudioside A in combination, the present disclosure provides a sweetener composition for alleviating diabetes, which may allow a very slow digestion of glucose in the body to provide a gradual increase in blood glucose level, thereby preventing hyperglycemic symptoms in which the blood glucose levels in diabetic patients are sharply increased, while preventing hypoglycemic symptoms fatal to diabetic patients by supplying sugars slowly but consistently and appropriately without excessively inhibiting the supply of sugars.

In addition, according to another embodiment, the present disclosure provides a sweetener composition, which includes D-psicose and a digestion-resistant maltodextrin in a specific ratio, and thus has outstanding properties in terms of alleviation of diabetes as compared with sweetener compositions prepared in other optional ingredient ratios. According to a further embodiment, the present disclosure provides a sweetener composition for alleviating diabetes, which can reduce calories while increasing sweetness and has an improved quality of sweetness by adding a specific high-intensity sweetener.

### Description of Drawings

Fig. 1 is a graph depicting the change of blood glucose in Experimental Example 1.
Fig. 2 is a graph depicting the change of the area under the curve of glucose in Experimental Example 2.

### Mode for Invention

Hereinafter, the present invention will be described in more detail. Descriptions of details apparent to those skilled in the art having ordinary knowledge in this technical field or relevant fields will be omitted herein.

The present invention provides a sweetener composition for use in alleviating diabetes containing D-psicose, a digestion-resistant maltodextrin and rebaudioside A. The term "digestion-resistant" means a property that digestion is not easily performed in the small intestine of the human body.

A polysaccharide refers to a saccharide formed by binding two or more monosaccharide units, namely, a saccharide higher than a disaccharide.

The digestion-resistant maltodextrin is a class of dietary fibers and refers to a polysaccharide that is indigestible in the body.

A high-intensity sweetener refers to a sweetener exhibiting high sweetness several to hundreds times that of sugar. Since rebaudioside A is used in the sweetener composition of the disclosure, the bitter taste, metallic taste and the like characteristic of high-intensity sweeteners such as steviol glycoside may be avoided, thereby providing a sweetener composition for alleviating diabetes, which has an excellent taste.

According to yet another embodiment, the sweetener composition for alleviating diabetes contains the digestion-resistant maltodextrin in an amount of 0.01 times to 200 times, preferably 0.01 times to 100 times, more preferably 0.1 times to 50 times the weight of D-psicose.

Further, the sweetener composition for alleviating diabetes contains rebaudioside A in an amount of 0.001 times to 2 times, preferably 0.001 times to 1.5 times, more preferably 0.001 times to 1 times the weight of D-psicose.

Within this content range, the sweetener composition may have advantages in that problems of sharp rise in blood glucose level, upon ingesting the sweetener composition, or hypoglycemic symptoms in which fasting blood glucose level is excessively declined, may be inhibited and the sweetener composition has a sweetness similar to that of sugar. Hereinafter, the present invention will be described in more detail with reference to Examples, Comparative Examples and Experimental Examples; Example 1 (without Rebaudioside A) is not according to the invention. These examples are provided for illustration only and are not to be in any way construed as limiting the present invention.

### Examples 1, 2 and Comparative Examples 1 to 3

### Preparation of sweetener composition

Sweetener compositions as listed in Table 1 were prepared.

In Comparative Example 1, 5 g of sugar was used. Sweetener compositions of Comparative Examples 2 and 3, and Examples 1 and 2 were prepared by formulating the components as listed in Table 1 such that the compositions exhibited sweetness similar to that of 5 g of sugar.

**TABLE 1**

| Raw material (g) | Manufacturer | Relative Sweetness per g of raw material | Com. Ex.1 | Com. Ex. 2 | Com. Ex. 3 | Ex. 1 | Ex. 2 |
|---|---|---|---|---|---|---|---|
| Sugar | CJ Cheiljedang | 1 | 5 | - | - | - | - |
| Erythritol | Zivogreen | 0.63 | - | 8 | - | - | - |
| D-psicose | CJ Cheiljedang | 0.56 | - | - | 9 | 8 | 4 |
| Digestion-resistant maltodextrin | Matsudani Korea Ltd. | 0.1 | - | - | - | 5.6 | 3.5 |
| Rebaudioside A | GLG | 200 | - | - | - | - | 0.015 |
| Relative sweetness of sweetener composition | | | 5 | 5.04 | 5.04 | 5.04 | 5.03 |

Among the sweetener compositions, erythritol used in Comparative Example 2 corresponds to a material that is substantially calorie-free and provides only sweetness without affecting blood glucose level. Rebaudioside A used in Example 2 is a steviol glycoside and is a natural high-intensity sweetener prepared by extracting components having a sweet taste from steviol glycosides.

### Experimental Example 1

### Measurement of changes in blood glucose

### (1) Preparation of test specimen for measuring blood glucose and method of intake

In order to measure changes in blood glucose level after consuming each sweetener composition prepared in Comparative Examples 1 to 3 and Examples 1 and 2, the following experiment was carried out for a normal group having a fasting glycemic index of 100 mg/dL or less and consisting of five males and five females in their twenties to forties.

The sweetener composition intake was conducted by providing subjects with an identical meal and then allowing them to drink coffee containing the sweetener composition.

The meals given to the subjects are as listed in Table 2.

**TABLE 2**

| Food materials | Amount used (g) |
|---|---|
| Bread | 75 |
| Ham | 20 |
| Lettuce | 20 |
| Strawberry jam | 20 |
| Crabstick | 30 |
| Cheddar cheese | 10 |

As shown in Table 2, the meal given to the subjects consisted of 75 g of bread, 20g of ham, 20 g of lettuce, 20 g of strawberry jam, 30 g of crabstick and 10 g of cheddar cheese. As a result of analysis using Korean food composition table (CanPro 3.0, The Korean Nutrition Society), it was found that the meal had a total caloric content of 356.4 kcal, which consisted of 59.57% of sugar, 18.14% of protein and 22.27% of lipid. After the meal, the subjects drank coffee made by mixing 1.6 g of sugar free coffee and the sweetener composition prepared in Comparative Examples 1 to 3 and Examples 1 and 2 in 200 g of hot water.

The coffee composition given to the subjects is listed in Table 3.

**TABLE 3**

| Materials (g) | Manufacturer | Com. Ex. 1 | Com. Ex. 2 | Com. Ex. 3 | Ex. 1 | Ex.2 |
|---|---|---|---|---|---|---|
| Coffee | Dongsuh Food | 1.6 | 1.6 | 1.6 | 1.6 | 1.6 |
| Sugar | CJ Cheiljedang | 5 | - | - | - | - |
| Erythritol | Zivogreen | - | 8 | - | - | - |
| D-psicose | CJ Cheiljedang | - | - | 9 | 8 | 4 |
| Digestion-resistant maltodextrin | Matsudani Korea Ltd. | - | - | - | 5.6 | 3.5 |
| Rebaudioside A | GLG | - | - | - | - | 0.015 |

### (2) Measurement of changes in blood glucose levels

In order to measure changes in blood glucose after the meal, blood glucose level before meal was checked, followed by providing the subjects with the meal and then coffee was given. Blood glucose was measured for 2 hours at intervals of 30 minutes.

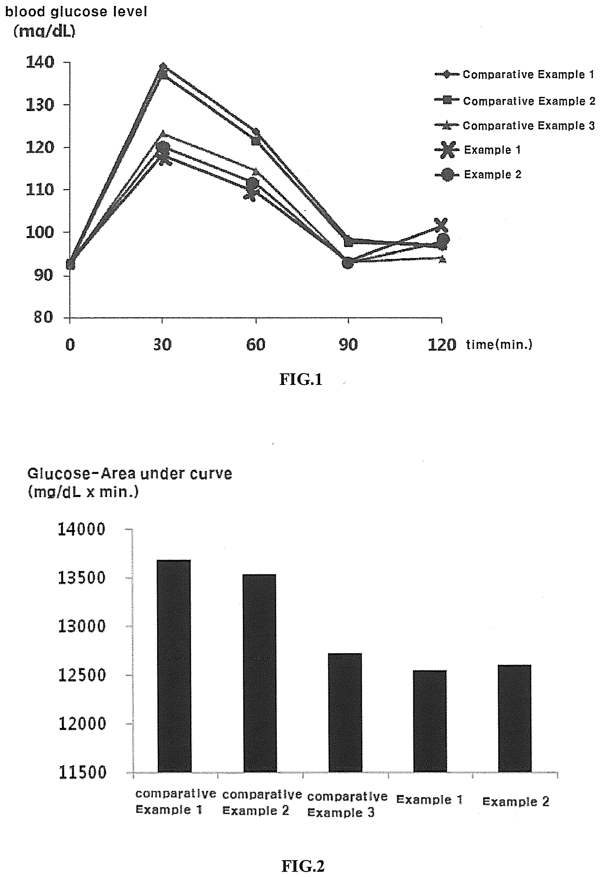

The changes in blood glucose level after meal and coffee intake are summarized in Table 4 (see Fig. 1).

**TABLE 4**

| | Time (min) | 0 (before meal) | 30 | 60 | 90 | 120 |
|---|---|---|---|---|---|---|
| Blood glucose (mg/dL) | Com. Ex.1 | 93.1 | 139.1 | 123.7 | 98.5 | 96.7 |
| | Com.Ex. 2 | 92.8 | 137.1 | 121.6 | 97.7 | 97.2 |
| | Com.Ex. 3 | 92.4 | 123.3 | 114.5 | 93.1 | 94.1 |
| | Ex. 1 | 92.9 | *118.3 | *109.5 | *93.4 | 101.6 |
| | Ex. 2 | 93.2 | *120.3 | *111.5 | *92.9 | 97.9 |

| | | | | | | |
|---|---|---|---|---|---|---|
| * corresponding to time zones in Examples 1 and 2 indicating significant difference of p<0.01 or less in blood glucose as compared with Comparative Example 1 (sugar). | | | | | | |

### Experimental Example 2

### Measurement of changes in area under the curve of blood glucose

The same experiment as in Experimental Example 1 was performed for 2 hours at an interval of 30 minutes to measure G-AUC (Glucose-Area under curve) of the subjects. The results of G-AUC are summarized in Table 5.

**TABLE 5**

| | Time (min) | 30 | 60 | 90 | 120 |
|---|---|---|---|---|---|
| G-AUC (mg/dL x min) | Comparative Example 1 | 3483 | 7425 | 10758 | 13686 |
| | Comparative Example 2 | 3449 | 7329 | 10619 | 13542 |
| | Comparative Example 3 | 3236 | 6803 | 9917 | 12725 |
| | Example 1 | 3168 | 6585 | 9629 | 12554 |
| | Example 2 | 3203 | 6680 | 9746 | 12608 |

As shown in Table 5, both Examples 1 and 2 showed a significant decrease in G-AUC for 120 minutes as compared with the composition of Comparative Example 1 (see Fig. 2).

As can be seen from the results of Experimental Examples 1 and 2, an increase in post-meal blood glucose persisted for about 60 minutes and 60 minutes post-meal, the blood glucose level showed a tendency to return to fasting blood glucose level.

When the subjects drank coffee containing the sweetener composition after the meal, increase in blood glucose to 60 minutes after drinking coffee was high in order of Comparative Example 1, Comparative Example 2, Comparative Example 3, Example 2, and Example 1. At 90 minutes to 120 minutes after having the meal and drinking the coffee, the blood glucose levels in Examples 1 and 2 were higher than in Comparative Examples 1 to 3.

One of the main causes for diabetes is a poor ability to control insulin, which leads to some problems. That is, a sharp rise in the blood glucose level after the meal occurs, causing excessive secretion of insulin, and the fasting blood glucose level causes hypoglycemic conditions.

Comparative Example 1 showed the highest increase in blood glucose resulting from the combined increase in blood glucose due to the meal and the coffee containing sugar. Comparative Example 2 showed increase in blood glucose due to the meal only. Comparative Example 3 showed that D-psicose has an effect of lowering increase in blood glucose after the meal.

It could be seen that the compositions of Examples 1 and 2 inhibited a sharp rise in post-meal blood glucose and showed an increase in fasting blood glucose as compared with the composition of the Comparative Examples. It can be understood that the composition of Example 1 provided an effect of preventing hypoglycemia on an empty stomach or after a certain period of time has elapsed from the meal by consistently supplying blood glucose while inhibiting a sharp change in blood glucose.

In other words, in Examples 1 and 2, it is determined that the carbohydrate absorption inhibition effect in the small intestine owing to D-psicose and the food digestion delay effect owing to the digestion-resistant maltodextrin may significantly lower a sharp rise in post-meal blood glucose, which allows a slow digestion of food, thereby consistently supply the body with saccharides even 2 hours after the meal.

### Experimental Example 3

### Sensory evaluation of sweetener composition

In order to evaluate the sensory perception of the sweetener compositions prepared in Examples 1 and 2, a sensory evaluation was performed on 25 adult males and females in their twenties to fifties using coffee each containing sucrose prepared in the same manner as in Experimental Example 1 (corresponding to Comparative Example 1 of Experimental Example 1) and coffee each containing the sweetener composition of Examples 1 and 2.

The sensory analysis was evaluated on a scale of 1 to 5. The results of the sensory analysis are summarized in Table 6.

**TABLE 6**

| | General acceptability | Color acceptability | General flavor acceptability | Taste acceptability | Aftertaste acceptability |
|---|---|---|---|---|---|
| Coffee containing sugar | 3.30 | 3.62 | 3.52 | 3.34 | 3.22 |
| Example 1 | 3.15 | 3.50 | 3.35 | 3.30 | 3.20 |
| Example 2 | 3.28 | 3.55 | 3.47 | 3.20 | 3.24 |

As can be seen from the results of Experimental Examples 1 to 3, it is determined that the sweetener composition of the present invention has sweetness and sensory quality similar to sugar and an effect of preventing a sharp rise in blood glucose and fasting hypoglycemic condition. Therefore, the sweetener composition corresponds to an effective sweetener for diabetic patients. Furthermore, the sweetener composition may be applied in various ways for persons requiring control/management of blood glucose.

## Claims

1. A sweetener composition for use in alleviating diabetes containing D-psicose, a digestion-resistant maltodextrin and rebaudioside A wherein the sweetener composition is to be administered after a meal.

2. The sweetener composition for the use of claim 1, wherein the digestion-resistant maltodextrin is present in an amount of 0.01 to 50 times the weight of D-psicose, and rebaudioside A is present in an amount of 0.001 to 1 times the weight of D-psicose.

## Patentansprüche

1. Süßstoffzusammensetzung zur Verwendung bei der Linderung von Diabetes, die D-Psicose, ein verdauungsresistentes Maltodextrin und Rebaudiosid A enthält, wobei die Süßstoffzusammensetzung nach einer Mahlzeit zu verabreichen ist.

2. Süßstoffzusammensetzung zur Verwendung nach Anspruch 1, wobei das verdauungsresistente Maltodextrin in einer Menge des 0,01- bis 50-fachen des Gewichtes der D-Psicose vorhanden ist und Rebaudiosid A in einer Menge des 0,001-bis 1-fachen des Gewichtes der D-Psicose vorhanden ist.

## Revendications

1. Composition d'édulcorant pour utilisation pour atténuer le diabète contenant du D-psicose, une maltodextrine résistante à la digestion et du rébaudioside A où la composition d'édulcorant est destinée à être administrée après un repas.

2. Composition d'édulcorant pour l'utilisation selon la revendication 1, dans laquelle la maltodextrine résistante à la digestion est présente dans une quantité de 0,01 à 50 fois la masse de D-psicose, et le rébaudioside A est présent dans une quantité de 0,001 à 1 fois la masse de D-psicose.
